(19) Europäisches Patentamt
European Patent Office
Office européen des brevets

(11) Veröffentlichungsnummer : **0 143 320**
**B1**

(12) # EUROPÄISCHE PATENTSCHRIFT

(45) Veröffentlichungstag der Patentschrift : **20.01.88**

(51) Int. Cl.⁴ : **C 07 C 118/00**, C 07 C 119/042

(21) Anmeldenummer : **84112671.7**

(22) Anmeldetag : **19.10.84**

(54) **Verfahren zur Herstellung von Isocyanaten durch thermische Spaltung von Urethanen.**

(30) Priorität : **29.10.83 DE 3339300**

(43) Veröffentlichungstag der Anmeldung : **05.06.85 Patentblatt 85/23**

(45) Bekanntmachung des Hinweises auf die Patenterteilung : **20.01.88 Patentblatt 88/03**

(84) Benannte Vertragsstaaten : **AT BE CH DE FR GB IT LI NL SE**

(56) Entgegenhaltungen :
**EP-A- 0 028 724**
**EP-A- 0 078 005**
**EP-A- 0 100 047**
**EP-A- 0 126 299**
**DE-A- 2 410 505**
**DE-A- 2 942 503**
**US-A- 2 409 712**
**US-A- 3 919 279**

(73) Patentinhaber : **BASF Aktiengesellschaft**
**Carl-Bosch-Strasse 38**
**D-6700 Ludwigshafen (DE)**

(72) Erfinder : **Mattner, Otto**
**Wormser Strasse 27a**
**D-6720 Speyer (DE)**
Erfinder : **Merger, Franz, Dr.**
**Max-Slevogt-Strasse 25**
**D-6710 Frankenthal (DE)**
Erfinder : **Towae, Friedrich, Dr.**
**Schwedlerstrasse 118**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 143 320 B1

**Beschreibung**

Die technische Herstellung von organischen Mono- und/oder Polyisocyanaten beruht allgemein auf der Phosgenierung der entsprechenden organischen Amine zu Carbamidsäurechloriden und deren thermische Spaltung, verbunden mit all den bekannten Nachteilen bezüglich Umweltschutz und Sicherheitsproblemen.

Nach einer anderen Herstellungsweise werden N-substituierte Urethane thermisch in der Gasphase oder in flüssiger Phase in Isocyanate gespalten. Bei der thermischen Spaltung treten jedoch vielfach zusätzlich verschiedene unerwünschte Nebenreaktionen auf. Genannt seien beispielsweise die Decarboxylierungsreaktion der Urethane, die von der Bildung primärer und sekundärer Amine sowie von Olefinen begleitet sein kann ; die Umsetzungen zwischen dem gebildeten Isocyanat und Urethan zu Allophanaten bzw. Amin zu Harnstoffen und die Polymerisation der Isocyanate zu Isocyanuraten.

Die Pyrolyse von Urethanen in der Dampfphase wird nach Angaben der DE-AS 19 44 719 (GB-PS 1 247 451) bei Temperaturen von 400 bis 600 °C in Gegenwart von Lewissäure als Katalysator durchgeführt, wobei das Isocyanat und der Alkohol durch fraktionierte Kondensation getrennt werden. 2,4-Toluylendiisocyanat wird z. B. durch Pyrolyse von Toluylen-2,4-diethylurethan in Gegenwart von Eisen-(III)-chlorid erhalten. Nachteile der Reaktion sind u. a. niedrige Ausbeuten, verbunden mit beträchtlichen Mengen eines polymeren Nebenproduktes, Zersetzung des Katalysators und Korrosion der Reaktionsapparatur. In der DE-OS 24 10 505 (US 3 870 739) wird ein Verfahren beschrieben, bei dem ein aromatisches Urethan bei einer Temperatur von 350 bis 550 °C und einem Druck von weniger als dem (m + 1) fachen des Isocyanatdampfdrucks in einer katalysatorfreien Pyrolysezone innerhalb von 15 Sekunden gespalten wird. Nachteilig an diesem Verfahren ist u. a., daß ein als Nebenprodukt anfallendes festes Polymer und dessen Abtrennung die Durchführung eines kontinuierlichen Verfahrens erschwert.

Die thermische Spaltung von Urethanen in flüssiger Phase wird beispielsweise in den DE-AS 24 21 503 (US 3 962 302) und DE-AS 25 30 001 (US 3 919 280) beschrieben. Nach Angaben der DE-AS 24 21 503 werden die Urethane in einem inerten Lösungsmittel, wie Alkylbenzolen, linearen und cyclischen Kohlenwasserstoffen und/oder Phthalsäureestern gelöst und unter Normal- oder Überdruck bei 175 bis 350 °C gespalten. Die Isolierung und Trennung des erhaltenen Isocyanats und Alkohols erfolgt mit Hilfe des Lösungsmittels als Trägermittel und/oder unter Verwendung eines Inertgases als Trägermittel. Gemäß DE-AS 25 30 001 werden als Reaktionsmedium höhermolekulare, gegebenenfalls substituierte aliphatische, cycloaliphatische oder aromatische Kohlenwasserstoffe, Ether, Ester oder Ketone verwendet. Zur Trennung der Spaltprodukte wird die Destillation genannt, wobei über Kopf Isocyanat, Alkohol und Trägermaterial abdestilliert werden, während das Reaktionsmedium als Bodenfraktion zurückbleibt.

Zur Herstellung von aromatischen Isocyanaten werden Urethane nach DE-OS 26 35 490 (US 4 081 472) bei Temperaturen von 150 bis 350 °C unter vermindertem Druck mit einer Lösung aus wenigstens einem Metallion, wie Ionen von Kupfer, Zink, Aluminium, Zinn, Titan, Vanadium, Eisen, Cobalt und Nickel als Katalysator, der in einem Lösungsmittel mit einem Siedepunkt von 200 °C in einer Metallkonzentration von wenigstens 0,001 Gew.%, bezogen auf das Lösungsmittel, gelöst ist, in Kontakt gebracht. Die Trennung der erhaltenen Spaltprodukte erfolgt durch fraktionierte Kondensation. Hierbei bleiben jedoch in geringen Mengen gebildete, nicht destillierbare Polymerisationsprodukte im Katalysator enthaltenden Lösungsmittelrückstand, wodurch nach einiger Zeit zusätzlich Reinigungsoperationen der die katalytisch wirksamen Metallionen enthaltenen Lösungsmittel notwendig werden.

Nach EP-PS 28 724 (US 4 330 479) erzielt man sehr gute Spaltergebnisse, wenn man Urethane an katalytisch wirksamen, oberflächenreichen Metallen, die in heterogener Phase vorliegen, spaltet. Nachteilig an diesem Verfahren ist, daß die als Katalysatoren eingesetzten Metalle mit der Zeit durch Belegung allmählich ihre katalytische Aktivität verlieren, wodurch ebenfalls zusätzlich Reinigungsoperationen notwendig werden.

Zur Beseitigung dieses Mangels wird nach Angaben der EP-OS 78 005 die thermische Spaltung in Gegenwart von Kohlenstoff, der vorzugsweise als Feststoffschüttung in einem Wirbelbett vorliegt, durchgeführt. Vorteilhaft is hierbei zwar, daß der durch polymere Neben- oder Zersetzungsprodukte desaktivierte Katalysator nicht regeneriert zu werden braucht, sondern durch Verbrennen rückstandsfrei und umweltfreundlich vernichtet werden kann, doch ist wegen der aufwendigen Wirbelbettechnik und den unvermeidlichen Reinigungsprozeduren für das Wirbelgut ein gewisser technischer Aufwand erforderlich.

Die Ausführungen zeigen, daß die bekannten Herstellungsverfahren für Isocyanate durch thermische Spaltung von Carbamidsäurechloriden oder N-substituierten Urethanen mehr oder weniger große Mängel aufweisen.

Aufgabe der vorliegenden Erfindung war es daher, ein Verfahren zur thermischen Spaltung von Urethanen in die entsprechenden Isocyanate zu entwickeln, bei dem die bei den bekannten Verfahren bestehenden Mängel beseitigt oder zumindest verringert werden.

Überraschenderweise wurde gefunden, daß man Urethane auf einfache Weise und mit sehr guten Ausbeuten thermisch spalten kann, wenn man sie, gegebenenfalls in Gegenwart von Lösungsmitteln, in flüssiger Phase unter Druck erhitzt und über eine bei erhöhtem Druck abspritzende Düse in einen evakuierten, Füllkörper enthaltenden Spaltreaktor eindüst.

Gegenstand der Erfindung ist somit ein Verfahren zur Herstellung von Isocyanaten durch thermische Spaltung von Urethanen in der Gasphase, wobei man die Urethane in flüssiger Phase über eine Düse in einen verminderten Druck aufweisenden, mit temperaturbeständigen, inerten Füllkörpern beschichteten Spaltreaktor eindüst und katalysatorfrei bei Temperaturen von 175 bis 600 °C spaltet, das dadurch gekennzeichnet ist, daß man

(a) die Urethane unter einem Druck von 20 bis 300 bar auf Temperaturen von 100 bis 400 °C erhitzt und

(b) über eine Düse, deren Abspritzdruck bei 20 bis 300 bar liegt, in den Spaltreaktor eindüst, wobei

(c) der Reaktor mit 10 bis 1 000 Urethanäquivalenten pro Liter Reaktorvolumen und Stunde beaufschlagt wird.

Im Vergleich zu bisher bekannten Methoden werden nach dem erfindungsgemäßen Verfahren Isocyanate in sehr guten Ausbeuten in technisch weitaus einfacherer Weise erhalten, da keine Katalysatoren und gegebenenfalls Lösungsmittel benötigt werden, die regeneriert oder ausgeschleust werden müssen, und weiterhin keine Apparaturen zur Urethan-Verdampfung vorgeschaltet sind, die wegen unvermeidlicher Zersetzungsreaktionen nach einiger Zeit gereinigt werden müssen. Eine vorgeschaltete Urethan-Verdampfung ist nicht notwendig, da die in flüssiger Phase unter Druck vorerhitzten Urethane beim Einsprühen in den evakuierten Spaltreaktor spontan in Isocyanat und Alkohol gespalten und die Spaltprodukte aus dem Spaltofen abgezogen werden.

Die Urethane können in Abwesenheit von Lösungsmitteln gespalten werden. Nach einer bevorzugten Ausführungsform werden die Urethane jedoch mit inerten Lösungsmitteln vermischt. Sofern inerte Lösungsmittel eingesetzt werden, kommen diese in Mengen von 5 bis 50 Gew.-Teilen, vorzugsweise 10 bis 30 Gew.-Teilen pro 100 Gew.-Teilen Urethan zur Verwendung.

Die Urethane werden, gegebenenfalls in Gegenwart der inerten Lösungsmittel, unter einem Druck von 20 bis 300 bar, vorzugsweise 50 bis 200 bar auf Temperaturen von 100 bis 400 °C, vorzugsweise 130 bis 350 °C, erhitzt und über eine Düse, deren Abspritzdruck bei 20 bis 300 bar, vorzugsweise bei 50 bis 200 bar liegt, in den Spaltreaktor eingedüst, wobei der Reaktor mit 10 bis 1000, vorzugsweise 100 bis 500 Urethanäquivalenten pro Liter Reaktorvolumen und Stunde beaufschlagt wird.

Sofern die Urethanspaltung in Gegenwart von inerten Lösungsmitteln durchgeführt wird, werden bevorzugt solche verwendet, deren Siedepunkt zwischen denen der entstehenden Isocyanate und Alkohol liegt. Beispielhaft genannt seien : aliphatische Kohlenwasserstoffe, wie die höheren Alkane Decan, Undecan, Dodecan, Tridecan, Tetradecan, Pentadecan, Hexadecan, Heptadecan, Dekalin, flüssiges Paraffin, alicyclische Kohlenwasserstoffe, wie Erdölfraktionen der Naphthenreihe ; gegebenenfalls substituierte aromatische Kohlenwasserstoffe, wie z. B. Naphthalin, 1- und 2-Methylnaphthalin, 1,2-, 1,4-, 1,6-, 2,7-, 2,6- und 2,3-Dimethylnaphthalin, 1-Ethylnaphthalin, Phenylnaphthalin, Benzylnaphthalin, Toluol, 1,2-, 1,3- und 1,4-Dimethylbenzol, 1,2,4- und 1,3,5-Trimethylbenzol, 1,2,3,5- und 1,2,4,5-Tetramethylbenzol, 1,3,5-Triethylbenzol, Hexyl-, Heptyl-, Octyl-, Nonyl-, Decylbenzol, Hexaethylbenzol, Diphenyl, 4,4'-Dimethyldiphenyl, Dibenzyl, Diphenylmethan und 4,4'-Dimethyl-diphenylmethan, halogensubstituierte aromatische Kohlenwasserstoffe, wie z. B. Chlorbenzol, 1,2- und 1,4-Dichlorbenzol, 1,2,3,4-, 1,2,3,5- und 1,2,4,5-Tetrachlorbenzol, Pentachlorbenzol, Ether, wie z. B. Diethylenglykol-dimethylether, Diethylenglykoldiethylether, Diisoamylether, Di-n-amylether, Dibenzylether, Diphenylether, γ-Methylnaphthylether und β-Ethylnaphthylether.

Besonders bewährt haben sich und daher vorzugsweise verwendet werden gegebenenfalls substituierte aromatische Kohlenwasserstoffe wie Benzol, Toluol, 1,2-, 1,3- und 1,4-Dimethylbenzol, 1,2,4- und 1,3,5-Trimethylbenzol, 1,2,3,5- und 1,2,4,5-Tetramethylbenzol, 1,3,5-Triethylbenzol, Hexyl-, Heptyl-, Octyl-, Nonyl-, Decylbenzol, Ether, wie z. B. Diethylenglykol-dimethylether, Diethylenglykoldiethylether, Diisoamylether, Di-n-amylether und Alkane, wie Cyclohexan, Heptan, Octan, Nonan, Decan, Undecan, Dodecan, Tridecan, Tetradecan, Pentadecan, Hexadecan, Heptadecan, Tetralin, Dekalin oder Gemische davon.

Urethane, die nach dem erfindungsgemäßen Verfahren thermisch in Isocyanat und Alkohol gespalten werden, besitzen die allgemeine Formel

$$R(NHCOOR')_n,$$

in der bedeuten :

R einen heterocyclischen, aliphatischen, cycloaliphatischen, aromatischaliphatischen oder aromatischen Rest mit 1 bis 60 Kohlenstoffatomen, vorzugsweise 4 bis 20 Kohlenstoffatomen, insbesondere einen gegebenenfalls substituierten Aryl- und/oder Alkylrest, wobei der Alkylrest linear, verzweigt oder cyclisch sein kann und gegebenenfalls noch Heteroatome wie z. B. Schwefel, Sauerstoff oder Stickstoff und der Arylrest Methylgruppen als Brückenglieder gebunden enthalten kann,

R' einen gegebenenfalls substituierten aliphatischen Rest mit 1 bis 20 Kohlenstoffatomen, vorzugsweise 1 bis 10 Kohlenstoffatomen, einen aromatischen oder aromatisch-aliphatischen Rest mit 6 bis 20 Kohlenstoffatomen, vorzugsweise 6 bis 15 Kohlenstoffatomen oder einen cycloaliphatischen Rest mit 3 bis 15 Kohlenstoffatomen, vorzugsweise 3 bis 7 Kohlenstoffatomen und

n eine ganze Zahl von 1 bis 6, vorzugsweise 1 bis 3 und insbesondere 1 bis 2.

Als Reste R seien im Falle der gegebenenfalls substituierten Arylurethane beispielsweise genannt die Reste von aromatischen Monoaminen, wie Anilin, substituierten Anilinen, wie in 2-, 3- und/oder 4-Stellung

durch Nitro-, Methyl-, Ethyl-, n-Propyl-, Isopropyl-, n-Butyl-, Isobutyl-, sek.-Butyl-, tert.-Butylgruppe oder ein Chloratom substituierten Anilinen, ortho-, meta- und/oder para-Hydroxi-, Methoxi-, Ethoxi-, Propoxi-, Isopropoxi-, N-Butoxi-, Isobutoxi-, sek.-Butoxi- und tert.-Butoxianilin ; durch eine Aminogruppe in m- und/oder p-Stellung substituierte Benzoesäurealkylester mit 1 bis 4 Kohlenstoffatomen im Alkylrest, durch eine Aminogruppe in m- und/oder p-Stellung substituierte N-Alkoxicarbonylaminobenzole und -toluole mit 1 bis 4 Kohlenstoffatomen im Alkylrest ; α- und β-Naphthylamin ; aromatischen Diaminen wie 1,3- und 1,4-Diaminobenzol ; durch eine Nitro-, Methyl-, Ethyl-, n-Propyl-, Isopropyl-, n-Butyl-, Isobutyl-, sek.-Butyl-, tert.-Butyl-, Methoxi-, Ethoxi-, n-Propoxi-, Isopropoxi-, n-Butoxi-, Isobutoxi-, sek.-Butoxi-, tert.-Butoxigruppe oder ein Halogenatom, vorzugsweise ein Fluor- und/oder Chloratom, in 2- und/oder 4-Stellung substituiertem 1,3-Diaminobenzol oder in 2-Stellung substituiertem 1,4-Diaminobenzol, 1,5- und 1,8-Diaminonaphthalin, 4,4'-Diaminodiphenyl, 2,2'-, 2,4'- und 4,4'-Diamino-diphenylmethan und den entsprechenden Isomerengemischen und aromatischen Polyamine, wie 1,3,5-Triaminobenzol, 2,4,6-Triaminotoluol und 1,3,5-Triaminonaphthalin.

Weiterhin im Fall der gegebenenfalls substituierten Alkylurethane die Reste von aliphatischen Monoaminen wie Methylamin, Ethylamin, Propylamin, Isopropylamin, n-Butylamin, sek.-Butylamin, tert.-Butylamin, Isobutylamin, 2- und 3-Methylbutylamin, Neopentylamin, n-Pentylamin, 2-Methyl-pentylamin, sek.-iso-Amylamin, n-Hexylamin, 2-Methylhexylamin, 2-Ethylhexylamin, n-Heptylamin, n-Octylamin, n-Nonylamin, n-Decylamin, n-Dodecylamin, 2-Phenylpropylamin, Benzylamin, Cyclopentylamin, Cyclohexylamin, tert.-Butylcyclohexylamin, von aliphatischen Diaminen wie Ethylen-diamin, 1,3- und 1,2-Propylendiamin, 2,2-Dimethyl-1,3-propylen-diamin, 1,4-Butylendiamin, 2-Ethyl-1,4-butylen-diamin, 1,5-Pentamethylen-diamin, 2-Methyl-1,5-pentamethylen-diamin, 1,6-Hexamethylen-diamin, 2,2,4-Trimethyl-1,6-hexamethylen-diamin, 1,8-Octamethylen-diamin, 1,10-Decylen-diamin, 1,12-Dodecylen-diamin und 1,4-Hexahydroxylylen-diamin, cycloaliphatische Diamine, wie 1,2-, 1,3- und 1,4-Cyclohexandiamin, 2,4- und 2,6-Hexahydrotoluylen-diamin sowie die entsprechenden Isomerengemische, aliphatischcycloaliphatische Diamine, wie 4,4'-, 2,4'- und 2,2'-Diamino-dicyclohexylmethan sowie die entsprechenden Isomerengemische, 2,2-Di-(4-aminocyclohexyl)-propan, 3-Aminomethyl-3,5,5-trimethylcyclohexylamin und Di-cyclopentadienylverbindungen der Formel

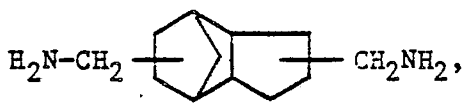

Heteroatome oder heterocyclische Reste gebunden enthaltende Diamine wie 3,3'-Diamino-dipropylether, gegebenenfalls substituierte N,N'-Bis-(aminoalkyl)-piperazin, z. B. N,N'-Di-(2,2-dimethyl-3-amino-propyl)-piperazin und N,N'-Bis-(aminopropyl)-piperazin.

Als typische Beispiele für Arylurethane, die nach dem erfindungsgemäßen Verfahren thermisch spaltbar sind, seien genannt : N-Phenyl-methylurethan, N-Phenyl-ethylurethan, 3,5-Dichlorphenyl-ethylurethan, 4-Methylphenylethylurethan, 2,4- und 2,6-Toluylen-di-methylurethan sowie die entsprechenden Isomerengemische, 2,4- und 2,6-Toluylen-di-ethylurethan, 2,4- und 2,6-Toluylen-di-butylurethan, 1,5-Naphthylen-di-ethylurethan, 4,4'-, 2,4'-, 2,2'-Methylen-diphenyl-dimethylurethan, 4,4'-, 2,4'- und 2,2'-Methylen-diphenyl-dibutylurethan, 4,4'-, 2,4'- und 2,2'-Methylendiphenyl-di-hexylurethan sowie die entsprechenden Isomerengemische.

Typische Beispiele für Alkylurethane sind : N-Methyl-octylurethan, N-Methyl-hexylurethan, N-Ethyldodecylurethan, N-Methyl-phenylurethan, N-Ethyl-phenylurethan, N-Propyl-phenylurethan, N-Propyl-decylurethan, N-Cyclohexyl-methylurethan, 1,6-Hexamethylen-dibutylurethan, 1,6-Hexamethylen-diethylurethan, 1,6-Hexamethylen-dimethylurethan, 2,2,4-Trimethyl-1,6-hexamethylen-dibutylurethan, 1,4-Hexahydroxylylen-diethylurethan, 1,4-Cyclohexyl-dimethylurethan, 1,4-Cyclohexyl-dibutylurethan und 3-(Butoxicarbonyl-aminomethyl)-3,5,5-trimethylcyclohexyl-butylurethan.

Die Spaltung der erhitzten Urethane erfolgt bereits beim Eindüsen in den Spaltreaktor bei Temperaturen von 175 bis 600 °C, vorzugsweise 300 bis 480 °C und insbesondere 360 bis 440 °C diskontinuierlich oder kontinuierlich bei einem Druck von gleich oder kleiner als 500 mbar, vorzugsweise 0,1 bis 200 mbar und insbesondere 1 bis 100 mbar.

Der Spaltreaktor, der im allgemeinen säulenförmig ist, kann einen Querschnitt in beliebiger Form, z. B. in Form eines Quadrats, einer Ellipse oder eines Kreises aufweisen. Vorzugsweise verwendet man langgestreckte, zylinderförmige Spaltreaktoren. Das Verhältnis von Innendurchmesser zu Länge des Spaltreaktors beträgt im allgemeinen 1 : 2 bis 1 : 100, vorzugsweise 1 : 10 bis 1 : 500. Die Spaltreaktoren können senkrecht oder waagrecht ausgerichtet sein und auch Zwischenlagen einnehmen. Vorzugsweise verwendet werden senkrecht stehende Röhrenöfen als Spaltreaktoren, bei denen der Rohrinnendurchmesser etwa 10 bis 100 mm und die Rohrlänge ungefähr 0,5 bis 5 m beträgt.

Erfindungsgemäß wird die Urethanspaltung in Gegenwart von thermisch stabilen Reaktorfüllkörpern durchgeführt. Als Füllkörper geeignet sind alle inerten, temperaturbeständigen und vorzugsweise gasdurchlässigen Materialien, wie z. B. Perlen, Wolle, Ringe und/oder Späne aus Kohle, Stahl, Messing, Kupfer, Zink, Aluminium, Titan, Chrom, Cobalt, Nickel und/oder Quarz. Einige dieser Materialien wie z. B. Stahl, Messing und Zink haben sich besonders bewährt und werden daher bevorzugt verwendet.

Aus dem Spaltreaktor führt man die in der Dampfphase befindlichen Dissoziationsprodukte, die

nahezu ausschließlich aus Isocyanat und Alkohol bestehen, in eine Zweistufen-Dampfkondensationsvorrichtung. Bei der in Beispiel 1 gezeigten Spaltung von 1,6-Hexamethylen-dibutylurethan wird beispielsweise bei einem Systemdruck von 20 bis 40 mbar im ersten Kondensator zweckmäßigerweise eine Kondensationstemperatur von 50 bis 100 °C und in der zweiten Kondensationsstufe zweckmäßigerweise eine Kondensationstemperatur von —10 bis 30 °C beim obengenannten Systemdruck eingestellt.

Alternativ ist es möglich, besonders bei Verwendung eines zusätzlichen Lösungsmittels, die Spaltgase in eine oder mehrere Trennkolonnen zu leiten, wobei sie, gegebenenfalls unter Benutzung eines Zwischensieders, destillativ getrennt werden. Die Zwischensieder sind im bevorzugten Fall mit den oben genannten Lösungsmitteln identisch.

Der anfallende Alkohol kann ohne vorhergehende Reinigung beispielsweise erneut zur Herstellung von Urethanen verwendet werden.

Das erhaltene Isocyanat wird zur Reinigung üblicherweise destilliert und besitzt danach eine Reinheit von über 99,5 Gew.%.

## Beispiel 1

Über eine Dosierkolbenpumpe mit beheiztem Pumpenkopf wurden aus einem auf 120 °C erwärmten Vorratsgefäß innerhalb 120 min 567 g einer Mischung aus 80 Gew.% Hexamethylen-dibutylurethan-1,6 und 20 Gew.% Dekalin in einen auf 430 °C erhitzten, mit 3 mm $V_2A$-Drahtnetzringen gefüllten Spaltreaktor aus $V_2A$-Stahl von 1 000 mm Länge und 40 mm Durchmesser eingedüst. Die Temperatur der Düse betrug 170 °C, ihr Abspritzdruck lag bei 150 bis 160 bar. Der Druck im Spaltreaktor betrug 14 mbar.

Die Spaltgase wurden in einer zweistufigen Kondensationseinrichtung fraktioniert, wobei in der ersten, bei 55 bis 60 °C betriebenen Kondensationsstufe 276,2 g einer Mischung aus 76,5 Gew.% Hexamethylen-diisocyanat-1,6,5,9 Gew.% 6-Butoxicarbonylamino-hexylisocyanat, 1,7 Gew.% Hexamethylendibutylurethan-1,6 und 15,7 Gew.% Dekalin erhalten wurde. In der zweiten, bei —5 °C betriebenen Kondensationsvorrichtung sammelten sich 284 g einer Mischung aus Butanol und Dekalin, die noch 8,6 Gew.% Hexamethylendibutylurethan-1,6 enthielt. Hieraus ergab sich ein Umsatz an Hexamethylendibutylurethan von 93,6% und eine Ausbeute an Hexamethylendiisocyanat-1,6, bezogen auf umgesetztes Urethan, von 93,6 Gew.%.

## Beispiel 2

Analog den Angaben von Beispiel 1 düste man während 120 min 550 g einer Mischung von 70 Gew.% Hexamethylen-dibutylurethan und 30 Gew.% einer Benzinfraktion mit Siedepunkten zwischen 155 und 180 °C in den auf 420 °C erhitzten, mit 3 mm $V_2A$-Drahtnetzringen gefüllten Spaltreaktor. Die Temperatur der Düse betrug 200 °C, ihr Abspritzdruck lag bei 200 bis 230 bar. Der Druck im Spaltreaktor betrug 17 mbar.

Die Spaltgase wurden in einer zweistufigen Kondensationseinrichtung fraktioniert, wobei in der ersten bei 55 bis 60 °C betriebenen Kondensationsstufe 245 g einer Mischung aus 71,7 Gew.% Hexamethylen-diisocyanat-1,6, 9 Gew.% 6-Butoxicarbonylamino-hexylisocyanat, 1,5 Gew.% Hexamethylen-dibutylurethan und 18,4 Gew.% Benzin erhalten wurden. In der zweiten, bei —7 °C betriebenen Kondensationsvorrichtung sammelten sich 278 g einer Mischung aus Butanol und Benzin, die noch 8,7 Gew.% Hexamethylen-dibutylurethan enthielt. Der Umsatz an Hexamethylen-dibutylurethan betrug demnach 92,7 %, die Ausbeute an Hexamethylen-diisocyanat-1,6 91,9 %.

## Beispiele 3 bis 5

Analog den Angaben von Beispiel 1 wurden die in der folgenden Tabelle genannten Urethane in Isocyanate gespalten.

(Siehe Tabelle Seite 6 f.)

Tabelle

| Beispiel | Urethan | Düse Temp. [°C] | Düse Abspritzdruck [bar] | Spalttemp. [°C] | Isocyanat | Umwandlung [%] | Ausbeute [%] |
|---|---|---|---|---|---|---|---|
| 3 | 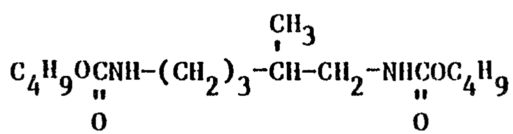 | 180-200 | 220-230 | 415 | 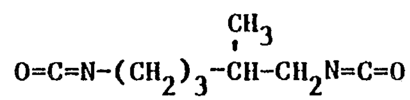 | 88,7 | 94,2 |
| 4 | 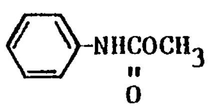 | 200-210 | 110-120 | 410 | 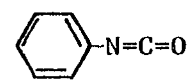 | 37 | 97,6 |
| 5 | 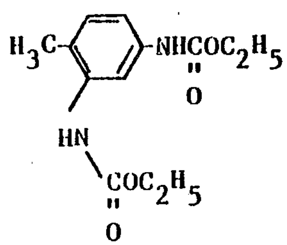 | 130-140 | 200-230 | 390 | 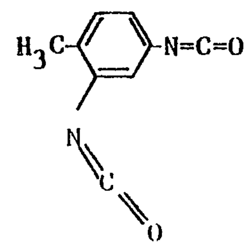 | 98,3 | 88,1 |

## Patentansprüche

1. Verfahren zur Herstellung von Isocyanaten durch thermische Spaltung von Urethanen in der Gasphase, wobei man die Urethane in flüssiger Phase über eine Düse in einen verminderten Druck aufweisenden, mit temperaturbeständigen, inerten Füllkörpern beschichteten Spaltreaktor eindüst und katalysatorfrei bei Temperaturen von 175 bis 600 °C spaltet, dadurch gekennzeichnet, daß man

(a) die Urethane unter einem Druck von 20 bis 300 bar auf Temperaturen von 100 bis 400 °C erhitzt und

(b) über eine Düse, deren Abspritzdruck bei 20 bis 300 bar liegt, in den Spaltreaktor eindüst, wobei

(c) der reaktor mit 10 bis 1 000 Urethanäquivalenten pro Liter Reaktorvolumen und Stunde beaufschlagt wird.

2. Verfahren nach Anspruch 1, dadurch gekennzeichnet, daß der verminderte Druck im Spaltreaktor gleich oder kleiner als 0,5 bar beträgt.

3. Verfahren nach Anspruch 1, dadurch gekennzeichnet, daß die temperaturbeständigen, inerten Füllkörper gasdurchlässig sind.

4. Verfahren nach Anspruch 1, dadurch gekennzeichnet, daß die katalysatorfreie Spaltung bei Temperaturen von 300 bis 480 °C und unter einem Druck von 0,1 bis 200 mbar durchgeführt wird.

5. Verfahren nach Anspruch 1, dadurch gekennzeichnet, daß man das zu spaltende Urethan mit einem inerten Lösungsmittel im Verhältnis von 100 : 5 bis 50 Gew.-Teilen versetzt.

6. Verfahren nach Anspruch 5, dadurch gekennzeichnet, daß das inerte Lösungsmittel einen Siedepunkt besitzt, der zwischen den Siedepunkten des bei der Spaltung entstehenden Isocyanates und Alkohols liegt.

7. Verfahren nach Anspruch 1, dadurch gekennzeichnet, daß die Urethane die allgemeine Formel

$$R(NHCOOR')_n$$

besitzen, in der bedeuten :

R einen heterocyclischen, aliphatischen, cycloaliphatischen, aromatisch-aliphatischen oder aromatischen Rest mit 1 bis 60 Kohlenstoffatomen,

R' einen gegebenenfalls substituierten aliphatischen Rest mit 1 bis 20 Kohlenstoffatomen, einen aromatischen oder aromatisch-aliphatischen Rest mit 6 bis 20 Kohlenstoffatomen oder einen cycloaliphatischen Rest mit 3 bis 15 Kohlenstoffatomen und

n eine ganze Zahl von 1 bis 6.

## Claims

1. A process for the preparation of an isocyanate by thermal cleavage of a urethane in the gas phase, the urethane, in the liquid phase, being sprayed via a nozzle into a cleavage reactor, which is under reduced pressure and is charged with heat-resistant, inert packing, and being cleaved in the absence of a catalyst at from 175 to 600 °C, wherein

(a) the urethane is heated to 100-400 °C under from 20 to 300 bar and

(b) sprayed into the cleavage reactor via a nozzle whose spray pressure is from 20 to 300 bar, and

(c) from 10 to 1,000 urethane equivalents per liter of reactor volume per hour are fed to the reactor.

2. A process as claimed in claim 1, wherein the reduced pressure in the cleavage reactor is less than or equal to 0.5 bar.

3. A process as claimed in claim 1, wherein the heat-resistant, inert packing is gas-permeable.

4. A process as claimed in claim 1, wherein the cleavage in the absence of a catalyst is carried out at from 300 to 480 °C and under from 0.1 to 200 mbar.

5. A process as claimed in claim 1, wherein the urethane to be cleaved is mixed with an inert solvent in a weight ratio of from 100 : 5 to 100 : 50.

6. A process as claimed in claim 5, wherein the inert solvent has a boiling point which is between the boiling points of the isocyanate and alcohol formed during the cleavage.

7. A process as claimed in claim 1, wherein the urethane is of the formula

$$R(NHCOOR')_n$$

where

R is a heterocyclic, aliphatic, cycloaliphatic, aromatic-aliphatic or aromatic radical of 1 to 60 carbon atoms,

R' is an unsubstituted or substituted aliphatic radical of 1 to 20 carbon atoms, an aromatic or aromatic-aliphatic radical of 6 to 20 carbon atoms or a cycloaliphatic radical of 3 to 15 carbon atoms and

n is an integer from 1 to 6.

**Revendications**

1. Procédé de préparation d'isocyanates par décomposition thermique d'uréthannes en phase gazeuse, dans lequel les uréthannes sont injectés en phase liquide, par une tuyère, dans un réacteur de décomposition garni de corps de remplissage inertes thermostables et maintenu à une pression réduite, dans lequel ils sont décomposés à des températures de 175 à 600 °C en l'absence de tout catalyseur, caractérisé en ce que

(a) les uréthannes sont chauffés sous une pression de 20 à 300 bars à des températures de 100 à 400 °C, puis

(b) injectés dans le réacteur de décomposition par une tuyère, dont la pression d'injection se situe entre 20 et 300 bars,

(c) le réacteur étant chargé de 10 à 1 000 équivalents uréthanne par litre de volume réactionnel et par heure.

2. Procédé suivant la revendication 1, caractérisé en ce que, dans le réacteur de décomposition, la pression réduite est égale ou inférieure à 0,5 bar.

3. Procédé suivant la revendication 1, caractérisé en ce que les corps de remplissage inertes thermostables sont perméables aux gaz.

4. Procédé suivant la revendication 1, caractérisé en ce que la décomposition en l'absence d'un catalyseur est réalisée entre 300 et 480 °C sous une pression de 0,1 à 200 millibars.

5. Procédé suivant la revendication 1, caractérisé en ce que l'uréthanne à décomposer est mélangé avec un solvant inerte dans une proportion de 100 : 5 à 50 parties en poids.

6. Procédé suivant la revendication 5, caractérisé en ce que le solvant inerte possède un point de fusion qui se situe entre le point de fusion de l'isocyanate et celui de l'alcool, formés lors de la décomposition.

7. Procédé suivant la revendication 1, caractérisé en ce que les uréthannes possèdent la formule générale

$$R(NHCOOR')_n,$$

dans laquelle

R désigne un groupe hétérocyclique, aliphatique, cycloaliphatique, aromatique-aliphatique ou aliphatique en $C_1$ à $C_{60}$,

R' représente un groupe aliphatique éventuellement substitué en $C_1$ à $C_{20}$, un groupe aromatique ou aromatique-aliphatique en $C_6$ à $C_{20}$ ou un groupe cycloaliphatique en $C_3$ à $C_{15}$ et

n est un nombre entier valant de 1 à 6.